(19) Europäisches Patentamt
European Patent Office
Office européen des brevets

(11) Veröffentlichungsnummer: **0 203 440**
**A2**

(12) # EUROPÄISCHE PATENTANMELDUNG

(21) Anmeldenummer: **86106399.8**

(22) Anmeldetag: **12.05.86**

(51) Int. Cl.[4]: **C 07 D 249/08**, C 07 D 233/60, A 01 N 43/653, A 01 N 43/50

(30) Priorität: **25.05.85 DE 3518916**

(43) Veröffentlichungstag der Anmeldung: **03.12.86 Patentblatt 86/49**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Krämer, Wolfgang, Dr., Rosenkranz 25, D-5093 Burscheid 2 (DE)**
Erfinder: **Steinbeck, Karl, Dr., Rosenkranz 36, D-5093 Burscheid 2 (DE)**
Erfinder: **Büchel, Karl Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen (DE)**
Erfinder: **Hänssler, Gerd, Dr., Am Arensberg 58a, D-5090 Leverkusen 3 (DE)**
Erfinder: **Reinecke, Paul, Dr., Steinstrasse 8, D-5090 Leverkusen 3 (DE)**

(54) **Dichlorcyclopropylalkyl-hydroxyalkyl-azol-Derivate.**

(57) Neue Dichlorcyclopropylalkyl-hydroxyalkyl-azol-Derivate der Formel

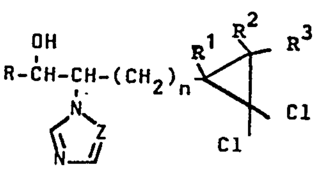

(I)

in welcher
R, $R^1$, $R^2$, $R^3$, Z und n die in der Beschreibung angegebene Bedeutung haben,
sowie deren Metallsalz-Komplexe und Säureadditions-Salze, ein Verfahren zur Hestellung der neuen Stoffe und deren Verwendung als Fungizide.
Neue Azolylketon-Derivate der Formel

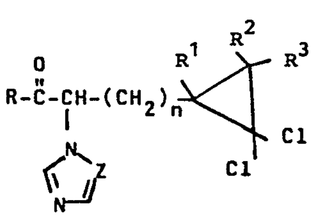

(II)

in welcher
R, $R^1$, $R^2$, $R^3$, Z und n die in der Beschreibung angegebene Bedeutung haben,
und ein Verfahren zur Herstellung der neuen Stoffe der Formel (II).

EP 0 203 440 A2

0203440
86106399.8

BAYER AKTIENGESELLSCHAFT 5090 Leverkusen, Bayerwerk
Konzernverwaltung RP
Patentabteilung Dü/by-c
Ia

Dichlorcyclopropylalkyl-hydroxyalkyl-azol-Derivate

Die vorliegende Erfindung betrifft neue Dichlorcyclopropylalkyl-hydroxyalkyl-azol-Derivate, ein Verfahren zu ihrer Herstellung sowie ihre Verwendung als Fungizide.

Es ist bereits bekannt geworden, daß bestimmte Triazolylhydroxyalkyl-Derivate und Triazolyl-keton-Derivate gute fungizide Eigenschaften besitzen (vgl. DE-OS 32 34 627). So können z.B. 1-Cyclopentyl-4,4-dimethyl-2-(1,2,4-triazol-1-yl)-pentan-3-ol, 1-Cyclohexyl-4,4-dimethyl-2-(1,2,4-triazol-1-yl)-pentan-3-ol, 4,4-Dimethyl-1-(2-methylcyclohexyl)-2-(1,2,4-triazol-1-yl)-pentan-3-ol und 4,4-Dimethyl-1-(4-methylcyclohexyl)-2-(1,2,4-triazol-1-yl)-pentan-3-ol und 1-Cycloheptyl-4,4-dimethyl-2-(1,2,4-triazol-1-yl)-pentan-3-on zur Bekämpfung von Pilzen verwendet werden. Die Wirkung dieser Stoffe ist jedoch, insbesondere bei geringen Aufwandmengen, nicht immer voll befriedigend.

Es wurden nun neue Dichlorcyclopropylalkyl-hydroxyalkyl-azol-Derivate der Formel

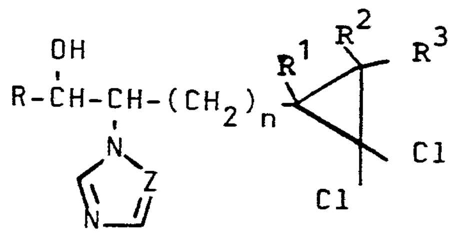

(I)

in welcher

R für gegebenenfalls substituiertes Cycloalkyl oder gegebenenfalls substituiertes Aryl sowie für die Gruppierungen

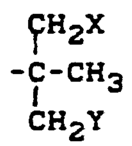 und 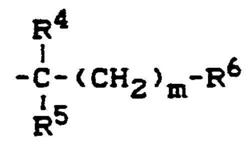 steht, wobei

X für Halogen steht,

Y für Wasserstoff oder Halogen steht,

$R^4$ und $R^5$ gleich oder verschieden sind und für Alkyl stehen,

$R^6$ für Alkyl, Halogenalkyl mit mehr als 2 Kohlenstoffatomen, Alkenyl, Alkinyl, gegebenenfalls substituiertes Cycloalkyl, gegebenenfalls substituiertes Aryl, gegebenenfalls substituiertes Aryloxy, gegebenenfalls substituiertes Arylthio oder für die Gruppierung

$R^7$-O-N=CH- steht,

in welcher

$R^7$ für Alkyl, Alkenyl oder gegebenenfalls substituiertes Benzyl steht,

und

m für die Zahlen 0, 1 oder 2 steht,

$R^1$,$R^2$ und $R^3$ unabhängig voneinander für Wasserstoff, Methyl oder Chlor stehen,

n für die Zahlen 1 oder 2 steht und

Z für ein Stickstoffatom oder die CH-Gruppe steht,

sowie deren Säureadditions-Salze und Metallsalz-Komplexe gefunden.

Die Verbindungen der Formel (I) besitzen mehrere asymmetrisch substituierte Kohlenstoffatome. Sie können deshalb in mehreren geometrischen Isomeren vorliegen, die in unterschiedlichen Mengenverhältnissen anfallen können. In allen Fällen liegen sie als Gemische optischer Isomeren vor. Die Erfindung betrifft sowohl die Isomerengemische als auch die einzelnen Isomeren.

Weiterhin wurde gefunden, daß man Dichlorcyclopropylalkyl-hydroxyalkyl-azol-Derivate der Formel (I) sowie deren

Säureadditions-Salze und Metallsalz-Komplexe erhält, wenn man Azolyl-keton-Derivate der Formel

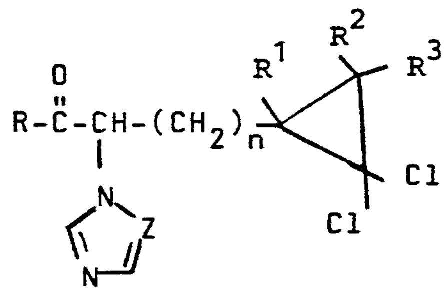

(II)

in welcher

R, $R^1$, $R^2$, $R^3$, n und Z die oben angegebenen Bedeutungen haben,

reduziert und gegebenenfalls anschließend eine Säure oder ein Metallsalz addiert.

Schließlich wurde gefunden, daß die neuen Dichlorcyclopropylalkyl-hydroxyalkyl-azol-Derivate der Formel (I) sowie deren Säureadditions-Salze und Metallsalz-Komplexe sehr gute fungizide Eigenschaften aufweisen.

Überraschenderweise zeichnen sich die erfindungsgemäßen Stoffe durch eine bessere fungizide Wirksamkeit aus als die aus dem Stand der Technik bekannten Triazol-Derivate 1-Cyclopentyl-4,4-dimethyl-2-(1,2,4-triazol-1-yl)-pentan-3-ol, 1-Cyclohexyl-4,4-dimethyl-2-(1,2,4-triazol-1-yl)-pentan-3-ol, 4,4-Dimethyl-1-(2-methylcyclohexyl)-2-(1,2,4-triazol-1-yl)-pentan-3-ol, 4,4-Dimethyl-1-(4-methylcyclohexyl)-2-(1,2,4-triazol-1-yl)-pentan-3-ol sowie 1-Cycloheptyl-4,4-dimethyl-2-(1,2,4-triazol-1-yl)-pentan-3-on, die konstitutionell und wirkungsmäßig naheliegende Verbindungen sind.

Die erfindungsgemäßen Dichlorcyclopropylalkyl-hydroxyalkyl-azol-Derivate sind durch die Formel (I) allgemein definiert. In dieser Formel stehen vorzugsweise

R für gegebenenfalls durch Alkyl mit 1 bis 3 Kohlenstoffatomen substituiertes Cycloalkyl mit 3 bis 7 Kohlenstoffatomen oder für Aryl mit 6 bis 10 Kohlenstoffatomen, wobei jeder dieser Aryl-Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, wie Fluor- und Chloratomen, Halogenalkoxy mit 1 oder 2 Kohlenstoffatomen und 1 bis 5 gleichen oder verschiedenen Halogenatomen, wie Fluor- oder Chloratomen, Halogenalkylthio mit 1 oder 2 Kohlenstoffatomen und 1 bis 5 gleichen oder verschiedenen Halogenatomen, wie Fluor- oder Chloratomen, Nitro, Cyano, Alkoxycarbonyl mit 1 bis 4 Kohlenstoffatomen im Alkoxyteil, Hydroximinoalkyl mit 1 oder 2 Kohlenstoffatomen, Alkoximinoalkyl mit 1 oder 2 Kohlenstoffatomen im Alkoxyteil und 1 oder 2 Kohlenstoffatomen im Alkylteil oder Phenyl, welches seinerseits durch Halogen und/oder Alkyl mit 1 bis 4 Kohlenstoffatomen substituiert sein kann, oder

R steht für die Gruppierungen

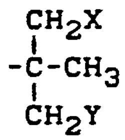 und 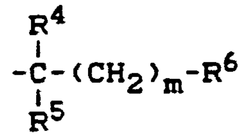 worin

X vorzugsweise für Fluor oder Chlor steht,

Y vorzugsweise für Wasserstoff, Fluor oder Chlor steht,

$R^4$ vorzugsweise für geradkettiges oder verzweigtes Alkyl mit 1 bis 4 Kohlenstoffatomen steht,

$R^5$ vorzugsweise für geradkettiges oder verzweigtes Alkyl mit 1 bis 4 Kohlenstoffatomen steht,

$R^6$ vorzugsweise für geradkettiges oder verzweigtes Alkyl mit 1 bis 4 Kohlenstoffatomen, für Halogenalkyl mit 2 oder 3 Kohlenstoffatomen und 1 bis 5 gleichen oder verschiedenen Halogenatomen, wie Fluor- und Chloratomen, für Alkenyl mit 2 bis 4 Kohlenstoffatomen, für Alkinyl mit 2 bis 4 Kohlenstoffatomen, für gegebenenfalls einfach bis dreifach, gleichartig oder verschieden durch Halogen, Alkyl mit 1 bis 4 Kohlenstoffatomen und/oder Alkoxy mit 1 bis 4 Kohlenstoffatomen substituiertes Cycloalkyl mit 3 bis 7 Kohlenstoffatomen, ferner für Phenyl, Phenoxy und Phenylthio steht, wobei jeder dieser Phenylreste einfach bis dreifach, gleichartig oder verschieden substituiert sein kann durch Halogen, Alkyl mit 1 bis 4 Kohlenstoffatomen, Alkoxy mit 1 bis

4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, wie Fluor- und Chloratomen, Halogenalkoxy mit 1 oder 2 Kohlenstoffatomen und 1 bis 5 gleichen oder verschiedenen Halogenatomen, wie Fluor- oder Chloratomen, Halogenalkylthio mit 1 oder 2 Kohlenstoffatomen und 1 bis 5 gleichen oder verschiedenen Halogenatomen, wie Fluor- oder Chloratomen, Nitro, Cyano, Alkoxycarbonyl mit 1 bis 4 Kohlenstoffatomen im Alkoxyteil, Hydroximinoalkyl mit 1 oder 2 Kohlenstoffatomen, Alkoximinoalkyl mit 1 oder 2 Kohlenstoffatomen im Alkoxyteil und 1 oder 2 Kohlenstoffatomen im Alkylteil oder Phenyl, welches seinerseits durch Halogen und/ oder Alkyl mit 1 bis 4 Kohlenstoffatomen substituiert sein kann, und $R^6$ außerdem für die Gruppierung

$R^7$-O-N=CH- steht,

in welcher

$R^7$ vorzugsweise für geradkettiges oder verzweigtes Alkyl mit 1 bis 4 Kohlenstoffatomen, Alkenyl mit 2 bis 4 Kohlenstoffatomen oder für Benzyl steht, welches im Phenylteil einfach bis dreifach, gleichartig oder verschieden substituiert sein kann durch Halogen, Alkyl mit 1 bis 4

Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, wie Fluor- und Chloratomen, Halogenalkoxy mit 1 oder 2 Kohlenstoffatomen und 1 bis 5 gleichen oder verschiedenen Halogenatomen, wie Fluor- oder Chloratomen, Halogenalkylthio mit 1 oder 2 Kohlenstoffatomen und 1 bis 5 gleichen oder verschiedenen Halogenatomen, wie Fluor- oder Chloratomen, Nitro, Cyano, Alkoxycarbonyl mit 1 bis 4 Kohlenstoffatomen im Alkoxyteil, Hydroximinoalkyl mit 1 oder 2 Kohlenstoffatomen, Alkoximinoalkyl mit 1 oder 2 Kohlenstoffatomen im Alkoxyteil und 1 oder 2 Kohlenstoffatomen im Alkylteil oder Phenyl, welches seinerseits durch Halogen und/oder Alkyl mit 1 bis 4 Kohlenstoffatomen substituiert sein kann, und

m für die Zahlen 0, 1 oder 2 steht,

$R^1$ für Wasserstoff oder Methyl,

$R^2$ für Wasserstoff, Methyl oder Chlor,

$R^3$ für Wasserstoff, Methyl oder Chlor,

n für die Zahlen 1 oder 2 und

Z für ein Stickstoffatom oder die CH-Gruppe.

Besonders bevorzugt sind diejenigen Verbindungen der Formel (I), in denen

R für gegebenenfalls durch Methyl und/oder Ethyl substituiertes Cycloalkyl mit 3 bis 6 Kohlenstoffatomen oder für Phenyl steht, das einfach bis dreifach, gleichartig oder verschieden substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, Ethoxycarbonyl, Hydroximinomethyl, 1-Hydroximinoethyl, Methoximinomethyl, 1-Methoximinoethyl oder Phenyl, welches seinerseits durch Fluor, Chlor und/oder Methyl substituiert sein kann, oder

R für die Gruppierungen

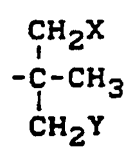 und 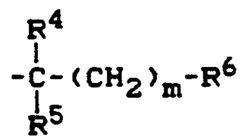 steht, worin

X für Fluor oder Chlor steht und

Y für Wasserstoff steht, oder

X und Y gleich sind und für Fluor oder Chlor stehen,

$R^4$ für Methyl oder Ethyl steht,

$R^5$ für Methyl oder Ethyl steht,

$R^6$ für Methyl, Ethyl, Isopropyl, Vinyl, Allyl, Propargyl, gegebenenfalls durch Chlor, Methyl, Ethyl und/oder Methoxy substituiertes Cyclopentyl, gegebenenfalls durch Chlor, Methyl, Ethyl und/oder Methoxy substituiertes Cyclohexyl oder für Phenyl, Phenoxy und Phenylthio steht, wobei jeder dieser Phenylreste einfach oder zweifach, gleichartig oder verschieden substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, Ethoxycarbonyl, Hydroximinomethyl, 1-Hydroximinoethyl, Methoximinomethyl, 1-Methoximinoethyl oder Phenyl, welches seinerseits durch Fluor, Chlor und/oder Methyl substituiert sein kann, und

$R^6$ außerdem für die Gruppierung

$R^7$-O-N=CH- steht,

in welcher

$R^7$ für Methyl, Ethyl, Propyl, Allyl, Propargyl oder für Benzyl steht, welches im Phenylteil einfach oder zweifach substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, Ethoxycarbonyl, Hydroximinomethyl, 1-Hydroximinoethyl, Methoximinomethyl, 1-Methoximinoethyl oder Phenyl, welches seinerseits durch Fluor, Chlor und/oder Methyl substituiert sein kann, und

m für die Zahlen 0 oder 1 steht,

$R^1$ für Wasserstoff oder Methyl steht,

$R^2$ für Wasserstoff, Methyl oder Chlor steht,

$R^3$ für Wasserstoff, Methyl oder Chlor steht,

n für die Zahlen 1 oder 2 steht und

Z für ein Stickstoffatom steht.

Besonders bevorzugt sind außerdem diejenigen Verbindungen der Formel (I), in denen

R für gegebenenfalls durch Methyl und/oder Ethyl substituiertes Cycloalkyl mit 3 bis 6 Kohlenstoffatomen oder für Phenyl steht, das einfach bis dreifach, gleichartig oder verschieden substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, Ethoxycarbonyl, Hydroximinomethyl, 1-Hydroximinoethyl, Methoximinomethyl, 1-Methoximinoethyl oder Phenyl, welches seinerseits

durch Fluor, Chlor und/oder Methyl substituiert sein kann, oder

R für die Gruppierungen

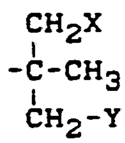 und 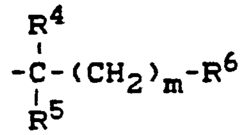 steht, worin

X für Fluor oder Chlor steht und

Y für Wasserstoff steht, oder

X und Y gleich sind und für Fluor oder Chlor stehen,

$R^4$ für Methyl oder Ethyl steht,

$R^5$ für Methyl oder Ethyl steht,

$R^6$ für Methyl, Ethyl, Isopropyl, Vinyl, Allyl, Propargyl, gegebenenfalls durch Chlor, Methyl, Ethyl und/oder Methoxy substituiertes Cyclopentyl, gegebenenfalls durch Chlor, Methyl, Ethyl und/oder Methoxy substituiertes Cyclohexyl oder für Phenyl, Phenoxy und Phenylthio steht, wobei jeder dieser Phenylreste einfach oder zweifach, gleichartig oder verschieden substituiert sein kann durch Fluor, Chlor, Brom, Methyl, Ethyl, Isopropyl, tert.-Butyl, Methoxy, Methylthio, Trifluormethyl, Trifluormethoxy,

Trifluormethylthio, Nitro, Cyano, Methoxycarbonyl, Ethoxycarbonyl, Hydroximinomethyl, 1-Hydroximinoethyl, Methoximinomethyl, 1-Methoximinoethyl oder Phenyl, welches seinerseits durch Fluor, Chlor und/oder Methyl substituiert sein kann, und

$R^6$ außerdem für die Gruppierung

$R^7$-O-N=CH- steht,

in welcher

$R^7$ für Methyl, Ethyl, Propyl, Allyl, Propargyl oder für Benzyl steht, welches im Phenylteil einfach oder zweifach substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, Ethoxycarbonyl, Hydroximinomethyl, 1-Hydroximinoethyl, Methoximinomethyl, 1-Methoximinoethyl oder Phenyl, welches seinerseits durch Fluor, Chlor und/oder Methyl substituiert sein kann, und

m für die Zahlen 0 oder 1 steht,

$R^1$ für Wasserstoff oder Methyl steht,

$R^2$ für Wasserstoff, Methyl oder Chlor steht,

$R^3$ für Wasserstoff, Methyl oder Chlor steht,

n für die Zahlen 1 oder 2 steht und

Z für eine CH-Gruppe steht.

Bevorzugte erfindungsgemäße Verbindungen sind auch Additionsprodukte aus Säuren und denjenigen Dichlorcyclopropylalkyl-hydroxyalkyl-azol-Derivaten der Formel (I), in denen R, $R^1$, $R^2$, $R^3$, n und Z die Bedeutungen haben, die bereits vorzugsweise für diese Substituenten bzw. den Index n genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure sowie Sulfonsäuren, wie z.B. p-Toluolsulfonsäure, 1,5-Naphthalindisulfonsäure und Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Verbindungen der Formel (I), in denen R, $R^1$, $R^2$, $R^3$, n und Z die Bedeutungen haben, die bereits vorzugsweise für diese Substituenten bzw. den Index n genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien diejenigen Dichlorcyclopropylalkyl-hydroxyalkyl-azol-Derivate genannt, die in der folgenden Tabelle 1 formelmäßig aufgeführt sind.

<u>Tabelle 1</u>

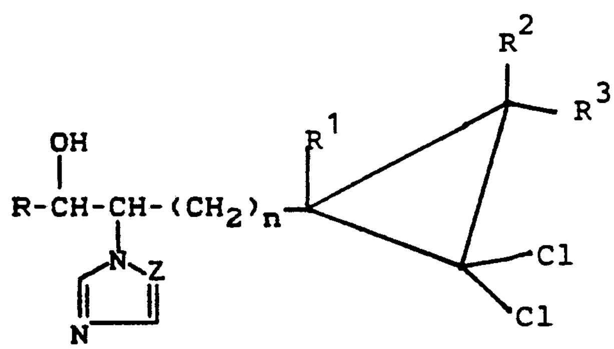

(I)

| R | Z | n | $R^1$ | $R^2$ | $R^3$ |
|---|---|---|---|---|---|
| 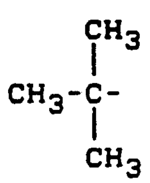 | N | 2 | H | H | H |
| 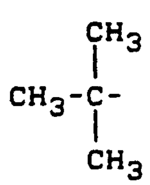 | " | 2 | " | $CH_3$ | $CH_3$ |

<u>Tabelle 1</u> (Fortsetzung)

| R | Z | n | $R^1$ | $R^2$ | $R^3$ |
|---|---|---|---|---|---|
| 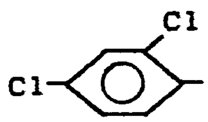 | N | 2 | H | H | H |
| " | " | " | " | $CH_3$ | $CH_3$ |
| 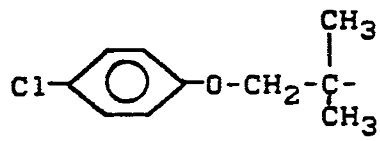 | " | " | " | H | H |
| " | " | " | " | $CH_3$ | $CH_3$ |
| " | -CH- | " | " | H | H |
| " | " | " | " | $CH_3$ | $CH_3$ |
| 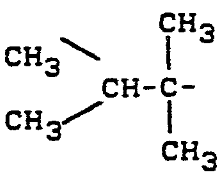 | N | " | " | " | " |
| 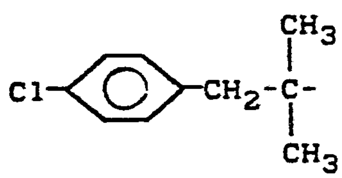 | " | " | " | H | H |
| " | " | " | " | $CH_3$ | $CH_3$ |
| 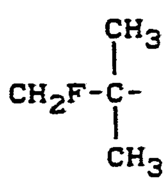 | N | 1 | H | H | H |
| 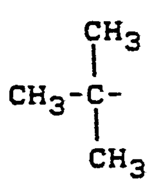 | N | 1 | $CH_3$ | H | H |

Tabelle 1 (Fortsetzung)

| R | Z | n | $R^1$ | $R^2$ | $R^3$ |
|---|---|---|---|---|---|
| 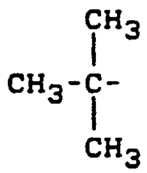 | N | 1 | H | H | $CH_3$ |
| 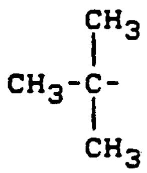 | N | 1 | H | Cl | $CH_3$ |

Verwendet man beispielsweise 1-(2,2-Dichlorcyclopropyl)-4,4-dimethyl-2-(1,2,4-triazol-1-yl)-pentan-3-on als Ausgangsstoff und Natriumborhydrid als Reduktionsmittel, so kann der Verlauf des erfindungsgemäßen Verfahrens durch das folgende Formelschema wiedergegeben werden:

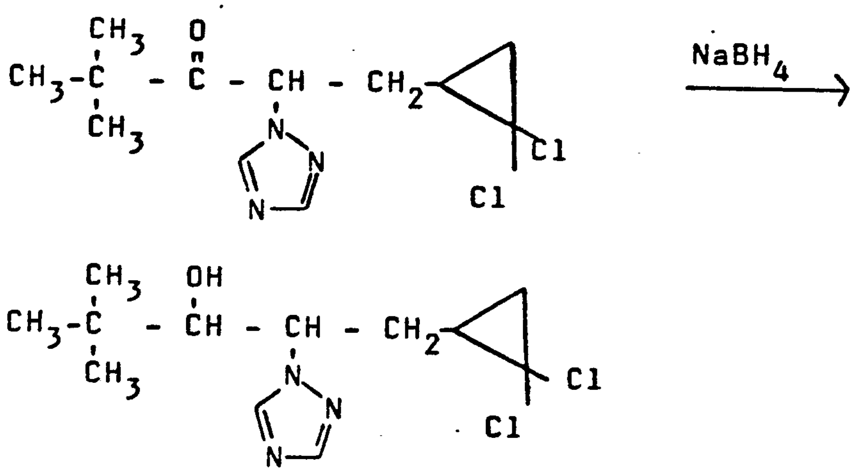


Die bei der Durchführung des erfindungsgemäßen Verfahrens als Ausgangsstoffe benötigten Azolyl-keton-Derivate sind

durch die Formel (II) allgemein definiert. In dieser Formel haben R, $R^1$, $R^2$, $R^3$, Z und n vorzugsweise diejenigen Bedeutungen, die bereits im Zusammenhang mit der Beschreibung der erfindungsgemäßen Stoffe der Formel (I) vorzugsweise für diese Substituenten bzw. diesen Index genannt wurden.

Als Beispiele für Ausgangsstoffe der Formel (II) seien diejenigen Azolyl-Keton-Derivate genannt, die in der folgenden Tabelle 2 formelmäßig aufgeführt sind.

<u>Tabelle 2</u>

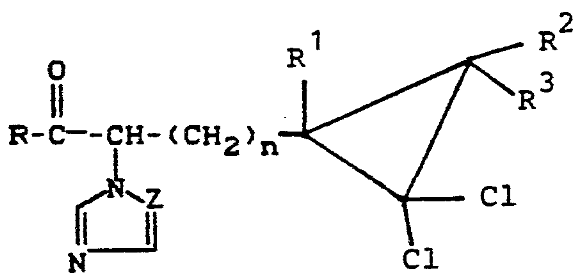

(II)

| R | Z | n | $R^1$ | $R^2$ | $R^3$ |
|---|---|---|---|---|---|
| 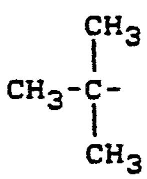 | N | 2 | H | $CH_3$ | $CH_3$ |
| 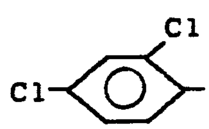 | N | 2 | H | H | H |
| " | " | " | " | $CH_3$ | $CH_3$ |
| 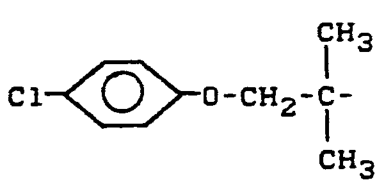 | " | " | " | H | H |
| " | " | " | " | $CH_3$ | $CH_3$ |
| " | -CH- | " | " | H | H |

Tabelle 2 (Fortsetzung)

| R | Z | n | $R^1$ | $R^2$ | $R^3$ |
|---|---|---|---|---|---|
| 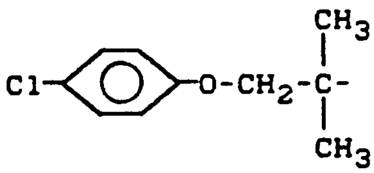 | -CH- | 2 | H | $CH_3$ | $CH_3$ |
| 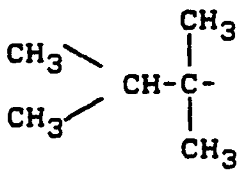 | N | " | H | $CH_3$ | $CH_3$ |
| 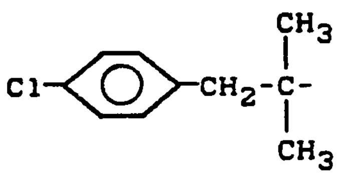 | " | " | " | H | H |
| " | " | " | " | $CH_3$ | $CH_3$ |
| 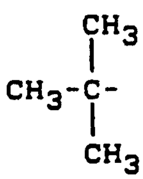 | " | " | $CH_3$ | " | " |
| " | N | 1 | H | H | $CH_3$ |
| " | N | 1 | H | Cl | $CH_3$ |

Die Azolylketon-Derivate der Formel (II) sind bisher noch nicht bekannt. Sie lassen sich herstellen, indem man Azolylketone der Formel

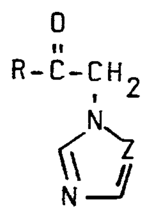

(III)

in welcher

R und Z die oben angegebene Bedeutung haben,

mit Verbindungen der Formel

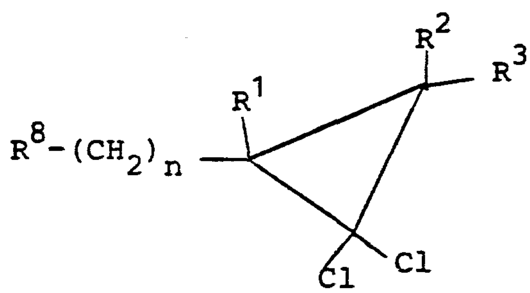

(IV)

in welcher

$R^1$, $R^2$, $R^3$ und n die oben angegebene Bedeutung haben und

$R^8$ für eine elektronenziehende Abgangsgruppe steht,

in Gegenwart eines inerten organischen Verdünnungsmittels, wie z.B. Dimethylsulfoxid, und in Gegenwart eines Säurebindemittels, wie z.B. Kaliumhydroxid, bei Temperaturen zwischen 0°C und 100°C umsetzt.

Die bei dem obigen Verfahren als Ausgangsstoffe benötigten Azolylketone der Formel (III) sind bekannt oder lassen sich nach prinzipiell bekannten Methoden herstellen (vgl. DE-OS 29 51 163 und DE-OS 30 48 266). So erhält man Azolylketone der Formel (III) z.B. dadurch, daß man Halogenketone der Formel

$$R-\overset{\overset{O}{\|}}{C}-CH_2-Hal \qquad (V)$$

in welcher

R die oben angegebene Bedeutung hat und

Hal für Chlor oder Brom steht,

mit 1,2,4-Triazol bzw. Imidazol in Gegenwart eines Verdünnungsmittels, wie beispielsweise Aceton, und in Gegenwart eines Säurebindemittels, wie beispielsweise Kaliumcarbonat, bei Temperaturen zwischen 20 und 150°C umsetzt.

Die Halogenketone der Formel (V) lassen sich herstellen, indem man Ketone der Formel

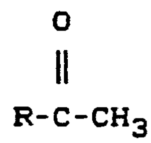

(VI)

in welcher

R die oben angegebene Bedeutung hat,

in einem inerten organischen Lösungsmittel bei Raumtemperatur mit Chlor oder Brom versetzt; oder z.B. mit üblichen Chlorierungsmitteln, wie Sulfurylchlorid, bei Temperaturen zwischen 20 bis 60°C umsetzt.

In den oben erwähnten Verbindungen der Formel (IV) haben $R^1$, $R^2$, $R^3$ und n vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. $R^8$ steht vorzugsweise für Halogen, wie z.B. Chlor oder Brom, oder für p-Methyl-phenyl-sulfonyloxy (= Tosyl).

Die Verbindungen der Formel (IV) sind teilweise bekannt (vgl. Liebigs Ann. Chem. 1979, 920 und Synthesis 1974, 274). Die bisher noch nicht bekannten Verbindungen der Formel (IV) lassen sich nach prinzipiell bekannten Verfahren synthetisieren. So erhält man Verbindungen der Formel (IV) z.B. dadurch, daß man Allyl-Derivate der Formel

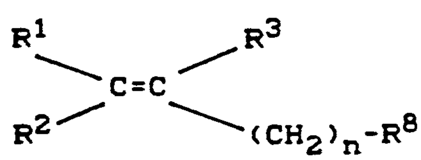

(VII)

in welcher

$R^1$, $R^2$, $R^3$, $R^8$ und n die oben angegebenen Bedeutungen haben,

mit Chloroform in Gegenwart von Triethylbenzylammoniumchlorid und Natronlauge bei Temperaturen zwischen 35 und 40°C umsetzt.

Die Ketone der Formel (VI) und die Allyl-Derivate der Formel (VII) sind allgemeine bekannte Verbindungen der organischen Chemie.

Bei der Durchführung des erfindungsgemäßen Verfahrens kommen als Substanzen zur Reduktion der Azolyl-keton-Derivate der Formel (II) alle üblichen Reagenzien in Betracht, die zur Reduktion derartiger Verbindungen geeignet sind. Vorzugsweise erfolgt die Umsetzung mit komplexen Hydriden gegebenenfalls in Gegenwart eines Verdünnungsmittels, oder mit Aluminiumisopropylat in Gegenwart eines Verdünnungsmittels.

Als komplexe Hydride können vorzugsweise Natriumborhydrid, Calciumborhydrid und Lithiumaluminiumhydrid eingesetzt werden.

Arbeitet man bei dem erfindungsgemäßen Verfahren mit komplexen Hydriden, so kommen als Verdünnungsmittel polare organische Lösungsmittel in Frage. Vorzugsweise verwendbar sind Alkohole, wie Methanol, Ethanol, Butanol und Isopropanol, und ferner Ether, wie Diethylether und Tetrahydrofuran.

Bei der Umsetzung mit komplexen Hydriden können die Reaktionstemperaturen innerhalb eines bestimmten Bereiches variiert werden. Im allgemeinen arbeitet man bei Temperaturen zwischen -10°C und +30°C, vorzugsweise zwischen 0°C und +20°C.

Bei der Durchführung der erfindungsgemäßen Reduktion mit komplexen Hydriden setzt man auf 1 Mol an Azolyl-keton-Derivat der Formel (II) etwa 1 Mol eines komplexen Hydrids ein. Die Isolierung der erfindungsgemäßen Verbindungen erfolgt in üblicher Weise. Im allgemeinen geht man so vor, daß man das Reaktionsgemisch ansäuert und gegebenenfalls nach vorheriger Neutralisation mit einem in Wasser wenig löslichen organischen Solvens extrahiert, und die organische Phase anschließend einengt.

Arbeitet man bei dem erfindungsgemäßen Verfahren mit Aluminiumisopropylat, so kommen als Verdünnungsmittel vorzugsweise Alkohole, wie Isopropanol, oder inerte Kohlenwasserstoffe, wie Benzol, in Frage.

Auch bei der Umsetzung mit Aluminiumisopropylat können die Reaktionstemperaturen innerhalb eines bestimmten Bereiches variiert werden. Im allgemeinen arbeitet man bei Temperaturen zwischen 20 und 120°C, vorzugsweise zwischen 50 und 100°C.

Bei der Durchführung der erfindungsgemäßen Reduktion mit Aluminiumisopropylat setzt man auf 1 Mol an Azolyl-keton-Derivat der Formel (II) etwa 1 bis 2 Mol Aluminiumisopropylat ein. Die Isolierung der erfindungsgemäßen Verbindungen erfolgt in üblicher Weise.

Zur Herstellung von Säureadditions-Salzen der Verbindungen der Formel (I) kommen alle diejenigen Säuren in Frage, die zu physiologisch verträglichen Salzen führen. Vorzugsweise verwendbar sind diejenigen Säuren, die bereits im Zusammenhang mit der Beschreibung der erfindungsgemäßen Stoffe als bevorzugt zu addierende Säuren genannt wurden.

Die Säureadditions-Salze der Verbindungen der Formel (I) lassen sich in einfacher Weise nach üblichen Salzbildungsmethoden herstellen. Im allgemeinen verfährt man so, daß man eine Verbindung der Formel (I) in einem geeigneten inerten Verdünnungsmittel löst und dann eine Säure hinzufügt. Die Isolierung erfolgt in bekannter Weise, zum Beispiel dadurch, daß man das Salz abfiltriert und gegebenenfalls durch Waschen mit einem inerten organischen Lösungsmittel reinigt.

Zur Herstellung von Metallsalz-Komplexen der Verbindungen der Formel (I) kommen vorzugsweise Salze von denjenigen Metallen in Betracht, die bereits im Zusammenhang mit der Beschreibung der erfindungsgemäßen Stoffe als bevorzugt zu addierende Metalle genannt wurden. Als Anionen dieser Metallsalze kommen vorzugsweise Halogenwasserstoffsäuren, wie zum Beispiel Chlorwasserstoffsäure und Bromwasserstoffsäure, ferner Phosphorsäure, Salpetersäure und Schwefelsäure in Frage.

Die Metallsalz-Komplexe von Verbindungen der Formel (I) lassen sich in einfacher Weise nach üblichen Methoden herstellen. Im allgemeinen geht man so vor, daß man ein

0203440

Metallsalz in Alkohol, wie zum Beispiel Ethanol, löst und dann eine Verbindung der Formel (I) hinzufügt. Die Isolierung erfolgt ebenfalls in bekannter Weise, zum Beispiel dadurch, daß man den Metallsalz-Komplex abfiltriert und gegebenenfalls durch Umkristallisation reinigt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;

Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Sphaerotheca-Arten, wie Sphaerotheca fuliginea an Gurken, Venturia-Arten, wie Venturia inaequalis an Äpfeln, Pyricularia-Arten, wie Pyricularia oryzae an Reis, und Fusarium-Arten, wie Fusarium culmorum an Weizen sowie auch zur Bekämpfung von Pyricularia an Reis, Drechslera graminea und Pseudocercosporella herpotrichiodes an Getreide eingesetzt werden. Besonders hervorzuheben ist, daß die erfindungsgemäßen Stoffe nicht nur eine protektive Wirkung haben, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die erfindungsgemäßen Wirkstoffe besitzen außerdem auch eine pflanzenwuchsregulierende Wirksamkeit.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie

synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemäßen Wirkstoffe geht aus den folgenden Beispielen hervor.

## Herstellungsbeispiele

### Beispiel 1

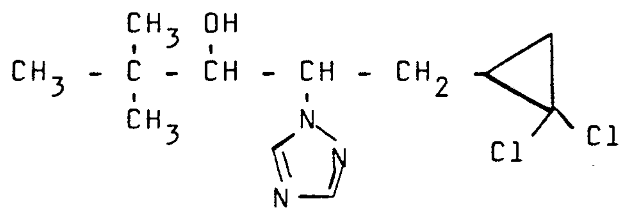

(I-1)

In eine Lösung von 13,63 g (0,047 Mol) 1-(2,2-Dichlorcyclopropyl)-4,4-dimethyl-2-(1,2,4-triazol-1-yl)-pentan-3-on in 100 ml Methanol werden 1,9 g (0,047 Mol) Natriumborhydrid unter Rühren bei 0°C portionsweise gegeben. Danach wird 4 Stunden bei Raumtemperatur nachgerührt und dann bei 0°C mit 20 ml 5n wäßriger Salzsäure versetzt. Das Reaktionsgemisch wird noch 3 Stunden bei Raumtemperatur gerührt und dann in 100 ml Eiswasser eingerührt. Es wird zweimal mit je 100 ml Methylenchlorid extrahiert, und die vereinigten organischen Phasen werden unter vermindertem Druck eingeengt.

Man erhält 13,0 g (95 % der Theorie) 1-(2,2-Dichlorcyclopropyl)-4,4-dimethyl-2-(1,2,4-triazol-1-yl)-pentan-3-ol als Diastereomerengemisch mit dem Brechungsindex $n_D^{20}$ = 1,5007.

Herstellung des Ausgangsproduktes

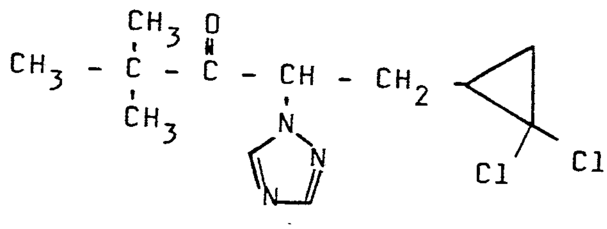

(II-1)

Eine Lösung von 8,4 g Kaliumhydroxid in 15 ml Wasser wird bei 0°C unter Rühren in eine Lösung von 25,05 g (0,15 Mol) 1-(1,2,4-Triazol-1-yl)-3,3-dimethyl-butan-2-on in 100 ml Dimethylsulfoxid gegeben. Danach werden bei 20°C unter Rühren 30,5 g (0,15 Mol) 1-Brommethyl-2,2-dichlorcyclopropan eingetropft. Man rührt weitere 4 Stunden bei 40°C nach, gibt das Reaktionsgemisch in 400 ml Eiswasser, extrahiert zweimal mit je 100 ml Methylenchlorid, wäscht die vereinigten organischen Phasen dreimal mit je 100 ml Wasser und zieht das Lösungsmittel unter vermindertem Druck ab. Dabei werden 42,1 g eines Produktes erhalten, das in 100 ml Essigester gelöst wird. Das entstehende Gemisch wird zweimal mit je 50 ml 2n wäßriger Salzsäure ausgeschüttelt. Die organische Phase wird eingeengt, und der verbleibende Rückstand wird in 50 ml mit Chlorwasserstoff gesättigtem Diethylether aufgenommen. Danach wird das Lösungsmittel unter vermindertem Druck abgezogen und der verbleibende Rückstand erneut in 200 ml Diethylether aufgenommen. Das Lösungsmittel wird dekantiert, der Rückstand in 100 ml Methylenchlorid aufgenommen und mit einer gesättigten, wäßrigen Natriumbicarbonatlösung (2 x 100 ml) ausgeschüttelt. Nach dem Abdestillieren des Methylenchlorids erhält man 14,3 g (32,9 % der Theorie) 1-(2,2-Dichlorcyclopropyl)-4,4-dimethyl-2-(1,2,4-triazol-1-yl)-pentan-3-on als Öl mit dem Brechungsindex $n_D^{20}$ = 1,500.

Beispiel 2

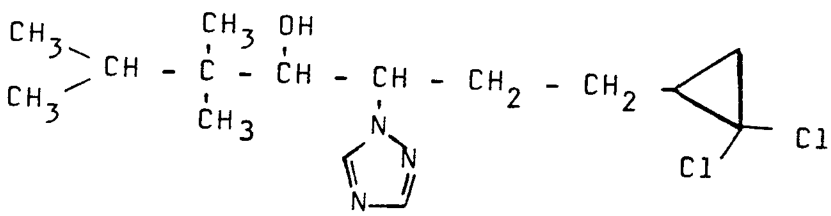
(I-2)

In eine Lösung von 2,32 g (0,007 Mol) 1-(2,2-Dichlorcyclopropyl)-5,5,6-trimethyl-3-(1,2,4-triazol-1-yl)-heptan-4-on in 30 ml Methanol werden 0,27 g (0,007 Mol) Natriumborhydrid unter Rühren bei 0°C portionsweise zugegeben. Danach wird 16 Stunden bei Raumtemperatur nachgerührt und dann bei 0°C mit 14 ml halbkonzentrierter wäßriger Salzsäure versetzt. Das Gemisch wird 6 Stunden bei Raumtemperatur gerührt und anschließend mit einer wäßrigen Natriumhydrogencarbonat-Lösung neutralisiert.

Es wird zweimal mit je 50 ml Methylenchlorid extrahiert, und die vereinigten organischen Phasen werden unter vermindertem Druck eingeengt.

Man erhält 2,3 g (99 % der Theorie) 1-(2,2-Dichlorcyclopropyl)-5,5,6-trimethyl-3-(1,2,4-triazol-1-yl)-heptan-4-ol als Diastereomerengemisch mit dem Brechungsindex $n_D^{23}$ : 1,5062.

Herstellung des Ausgangsproduktes

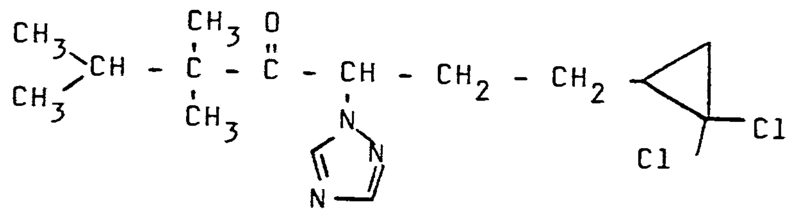
(II-2)

Eine Lösung von 1,5 g (0,026 Mol) Kaliumhydroxid in 1,5 ml Wasser wird bei 0°C unter Rühren in eine Lösung von 5,04 g (0,026 Mol) 1-(1,2,4-Triazol-1-yl)-3,3,4-trimethyl-pentan-2-on und 5,67 g (0,026 Mol) 1-(2-Bromethyl)-2,2-dichlorcyclopropan in 50 ml Dimethylsulfoxid gegeben. Die Mischung wird 16 Stunden bei Raumtemperatur gerührt und dann in 200 ml Wasser gegossen. Man extrahiert zweimal mit je 50 ml Methylenchlorid und wäscht die vereinigten organischen Phasen fünfmal mit je 50 ml Wasser. Der nach dem Abdestillieren des Lösungsmittels verbleibende Rückstand wird einer Kugelrohrdestillation unterworfen.

Man erhält 2,3 g (26,7 % der Theorie) 1-(2,2-Dichlorcyclopropyl)-5,5,6-trimethyl-3-(1,2,4-triazol-1-yl)-heptan-4-on als Öl vom Siedepunkt 150°C bei 0,1 mbar (Kugelrohr).

Nach der in den Beispielen 1 und 2 angegebenen Methode sowie den in der Beschreibung enthaltenen Angaben werden auch die in der folgenden Tabelle aufgeführten erfindungsgemäßen Verbindungen hergestellt.

**Tabelle 3**

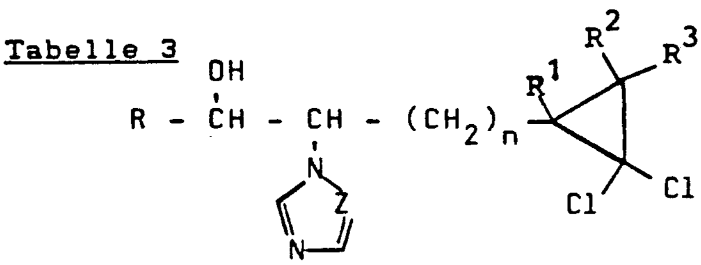

(I)

| Bsp. Nr. | R | Z | n | $R^1$ | $R^2$ | $R^3$ | Physikalische Konstante |
|---|---|---|---|---|---|---|---|
| 3 | 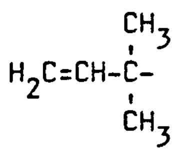 | N | 1 | H | $CH_3$ | $CH_3$ | $n_D^{20}$ =1,5040 |
| 4 | $(H_3C)_3C-$ | " | " | " | " | " | Fp=115-118°C |
| 5 | 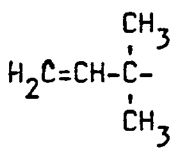 | " | " | " | H | H | $n_D^{20}$ =1,5083 |
| 6 | 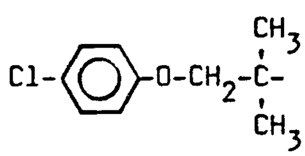 | " | " | $CH_3$ | " | " | amorphes Glas |
| 7 | 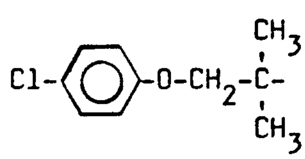 | " | " | H | $CH_3$ | $CH_3$ | Fp=151-157°C |
| 8 | 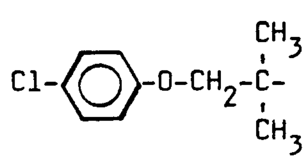 | " | " | " | H | H | Fp=178-188°C (HCl-Salz) |

Tabelle 3 (Fortsetzung)

| Bsp. Nr. | R | Z | n | $R^1$ | $R^2$ | $R^3$ | Physikalische Konstante |
|---|---|---|---|---|---|---|---|
| 9 | 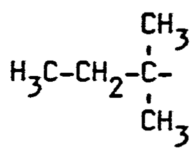 | N | 1 | H | H | H | $n_D^{20}$ =1,4787 |
| 10 | 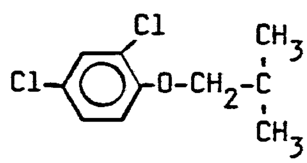 | " | " | " | " | " | Fp=62-78°C |
| 11 | 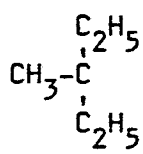 | " | " | " | " | " | Fp=113-116°C (HCl-Salz) |

Tabelle 3 (Fortsetzung)

| Bsp. Nr. | R | Z | n | $R^1$ | $R^2$ | $R^3$ | Physikalische Konstante |
|---|---|---|---|---|---|---|---|
| 12 | 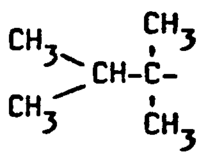 | N | 1 | H | H | H | amorphes Glas (HCl-Salz) |

Nach den Beispielen 1 und 2 angegebenen Methoden werden die nachfolgenden Ausgangsprodukte der Formel (II) erhalten:

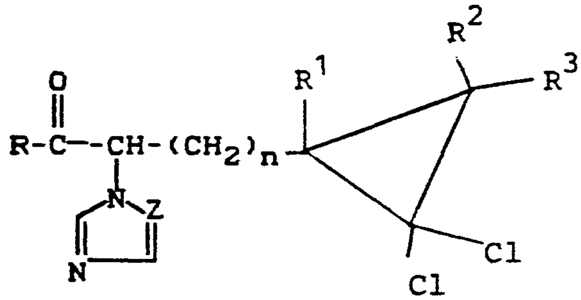

(II)

Tabelle 4

| Bsp. Nr. | R | Z | n | $R^1$ | $R^2$ | $R^3$ | Physikalische Konstante |
|---|---|---|---|---|---|---|---|
| 13 | 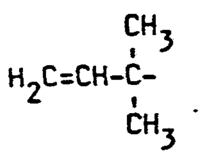 | N | 1 | H | $CH_3$ | $CH_3$ | $n_D^{20}$ =1,5060 |
| 14 | $(H_3C)_3C-$ | " | " | " | " | " | $n_D^{20}$ = 1,4995 |
| 15 | 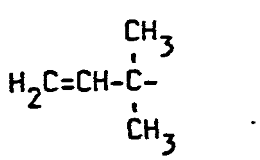 | " | " | " | H | H | $n_D^{20}$ =1,5056 |
| 16 | 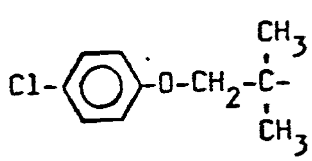 | " | " | $CH_3$ | " | " | $n_D^{20}$ =1,5327 |

Tabelle 4 (Fortsetzung)

| Bsp. Nr. | R | Z | n | $R^1$ | $R^2$ | $R^3$ | Physikalische Konstante |
|---|---|---|---|---|---|---|---|
| 17 | 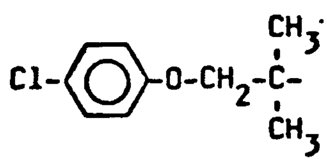 | " | " | H | $CH_3$ | $CH_3$ | $n_D^{20}$ =1,5291 |
| 18 | 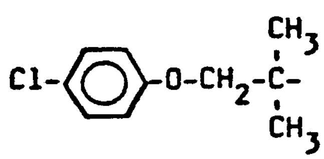 | " | " | " | H | H | amorph (HCl-Salz) |
| 19 | 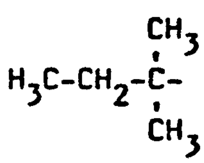 | N | 1 | H | H | H | $n_D^{20}$ =1,5083 |
| 20 | 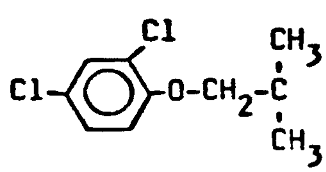 | " | " | " | " | " | zähes Oel |
| 21 | 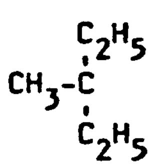 | " | " | " | " | " | $n_D^{20}$ =1,4918 |

Tabelle 4 (Fortsetzung)

| Bsp. Nr. | R | Z | n | $R^1$ | $R^2$ | $R^3$ | Physikalische Konstante |
|---|---|---|---|---|---|---|---|
| 22 | 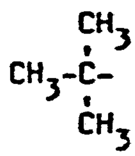 | N | 2 | H | H | H | $Kp_{0,1}$ = 150° C (Kugelrohr) |
| 23 | 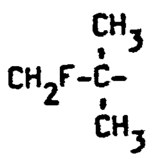 | N | 1 | H | H | H | zähes Oel |
| 24 | 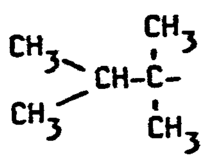 | " | " | " | " | " | zähes Oel |
| 25 | 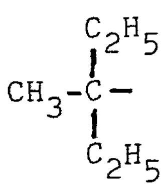 | " | " | " | " | " | $n_D^{20}$ = 1,5037 |

Die in der folgenden Tabelle aufgeführten Verbindungen der Formel (I) werden nach den in den Beispielen 1 und 2 angegebenen Methoden hergestellt.

Tabelle 5

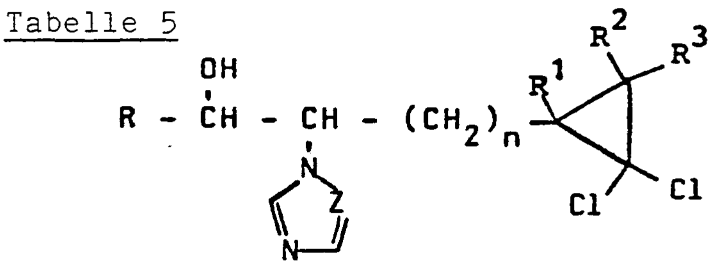 (I)

| Bsp. Nr. | R | Z | n | $R^1$ | $R^2$ | $R^3$ | Physikalische Konstante |
|---|---|---|---|---|---|---|---|
| 26 | 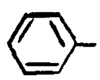 | N | 1 | H | H | H | $n_D^{20}$=1,5544 |
| 27 | 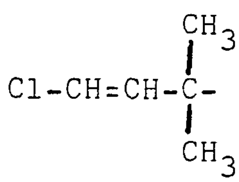 | N | 1 | H | H | H | $n_D^{20}$=1,5242 |
| 28 | 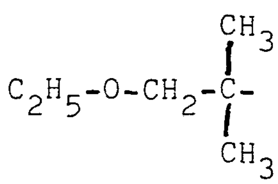 | N | 1 | H | H | H | $n_D^{20}$=1,4954 |
| 29 | 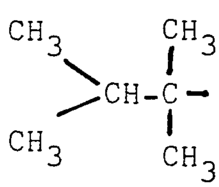 | N | 1 | H | H | H | (HCl-Salz) |
| 30 | 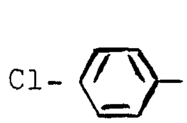 | N | 1 | H | H | H | NMR, $CDCl_3$ 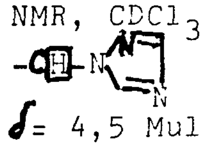 δ= 4,5 Multiplett |
| 31 | 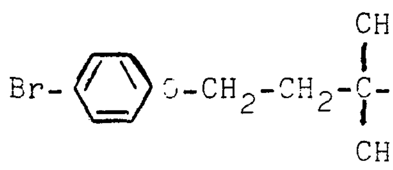 | N | 1 | H | H | H | Fp.=155-158°C (HCl-Salz) |

Tabelle 5 (Fortsetzung)

| Bsp. Nr. | R | Z | n | $R^1$ | $R^2$ | $R^3$ | Physikalische Konstante |
|---|---|---|---|---|---|---|---|
| 32 | 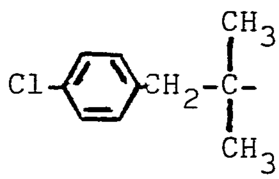 | N | 1 | H | H | H | NMR, $CDCl_3$ 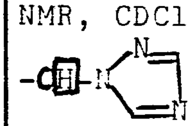 $\delta$=4,75 Multiplett |
| 33 | 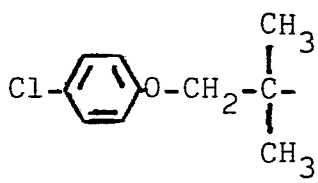 | N | 1 | $CH_3$ | H | H | Fp.=166-168°C |
| 34 | 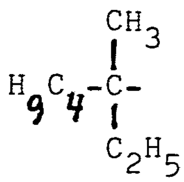 | N | 1 | H | H | H | $n_D^{20}$=1,4983 |
| 35 | 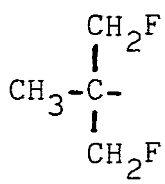 | N | 1 | H | H | H | $n_D^{20}$=1,4985 |
| 36 | 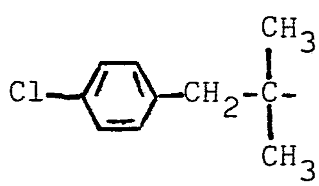 | N | 2 | H | $CH_3$ | $CH_3$ | Fp.=135-137°C |
| 37 | 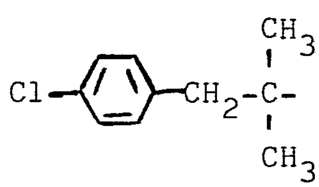 | N | 2 | H | H | H | Fp.=150-151°C |

Die in der folgenden Tabelle 6 aufgeführten Verbindungen der Formel (II) werden nach den in den Beispielen 1 und 2 angegebenen Methoden hergestellt.

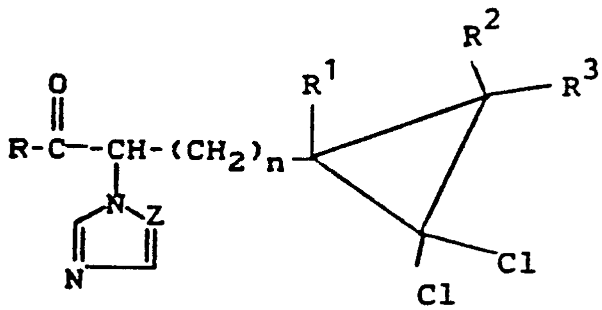

(II)

Tabelle 6

| Bsp. Nr. | R | Z | n | $R^1$ | $R^2$ | $R^3$ | Physikalische Konstante |
|---|---|---|---|---|---|---|---|
| 38 | 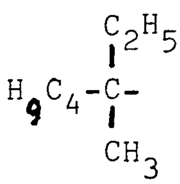 | N | 1 | H | H | H | Öl (HCl-Salz) |
| 39 | 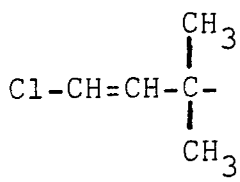 | N | 1 | H | H | H | $n_D^{20}$=1,5260 |
| 40 | 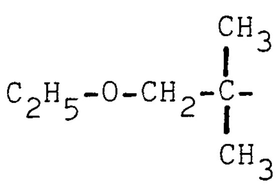 | N | 1 | H | H | H | $n_D^{20}$=1,4925 |
| 41 | 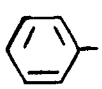 | N | 1 | H | H | H | Fp.=46-48°C |
| 42 | 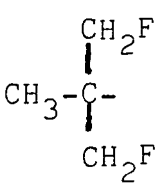 | N | 1 | H | H | H | $n_D^{20}$=1,4959 |

## Verwendungsbeispiele

In den folgenden Verwendungsbeispielen werden die nachstehend angegebenen Substanzen als Vergleichsverbindungen eingesetzt:

(A) = 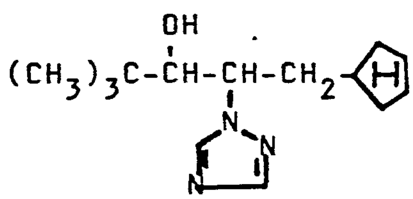

(B) = 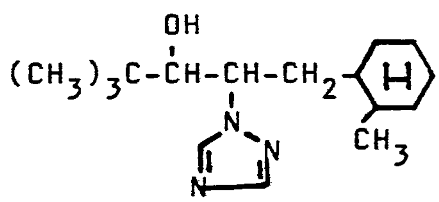

(C) = 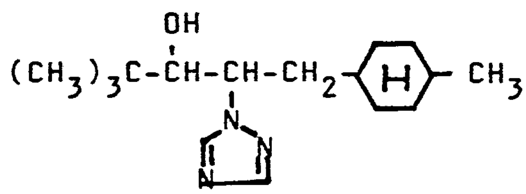

(D) = 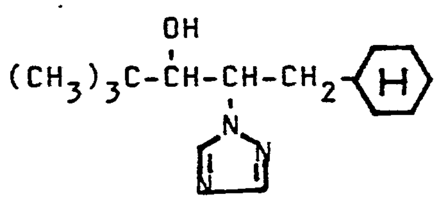

(E) = 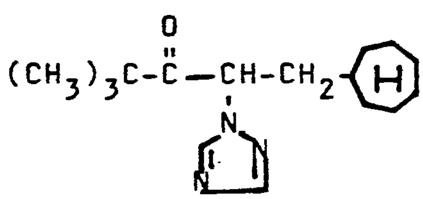

Beispiel A

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigen die in den Beispielen 1 und 4 beschriebenen erfindungsgemäßen Stoffe eine deutlich bessere Wirksamkeit als die Vergleichssubstanz (A).

Beispiel B

Sphaerotheca-Test (Gurke) / systemisch

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften wird die Wirkstoffzubereitung auf Einheitserde gegossen, in der sich junge, versuchsbereite Pflanzen befinden. 3 Tage nach der Behandlung werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigen die in den Beispielen 1 und 4 beschriebenen erfindungsgemäßen Stoffe eine deutlich bessere Wirksamkeit als die Vergleichssubstanzen (B) und (C).

0203440

Beispiel C

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

In diesem Test zeigen die in den Beispielen 1 und 4 beschriebenen erfindungsgemäßen Stoffe eine deutlich bessere Wirksamkeit als die Vergleichssubstanz (A).

Beispiel D

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

In diesem Test zeigen die in den Beispielen 1, 3, 4, 6 und 8 beschriebenen erfindungsgemäßen Stoffe eine deutlich bessere Wirkung als die Vergleichssubstanz (D).

## Beispiel E

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome.

In diesem Test zeigen die in den Beispielen 3,4,25,27,28 und 42 beschriebenen erfindungsgemäßen Stoffe eine deutlich bessere Wirkung als die Vergleichssubstanz (E).

0203440

## Patentansprüche

1. Dichlorcyclopropylalkyl-hydroxyalkyl-azol-Derivate der Formel

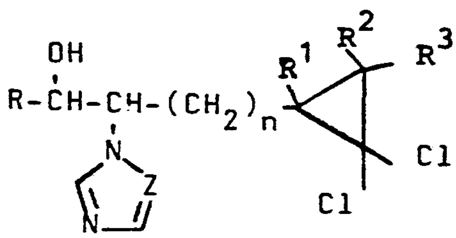

(I)

in welcher

R für gegebenenfalls substituiertes Cycloalkyl oder gegebenenfalls substituiertes Aryl sowie für die Gruppierungen

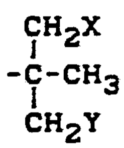

und

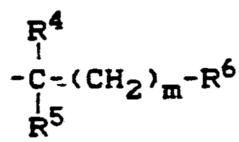

steht, wobei

X für Halogen steht,

Y für Wasserstoff oder Halogen steht,

$R^4$ und $R^5$ gleich oder verschieden sind und für Alkyl stehen,

$R^6$ für Alkyl, Halogenalkyl mit mehr als 2 Kohlenstoffatomen, Alkenyl, Alkinyl, gegebenenfalls substituiertes Cycloalkyl, gegebenenfalls substituiertes Aryl,

gegebenenfalls substituiertes Aryloxy, gegebenenfalls substituiertes Arylthio oder für die Gruppierung
$R^7$-O-N=CH- steht,

in welcher

$R^7$ für Alkyl, Alkenyl oder gegebenenfalls substituiertes Benzyl steht,

und

m für die Zahlen 0, 1 oder 2 steht,

$R^1$, $R^2$ und $R^3$ unabhängig voneinander für Wasserstoff, Methyl oder Chlor stehen,

n für die Zahlen 1 oder 2 steht und

Z für ein Stickstoffatom oder die CH-Gruppe steht,

sowie deren Säureadditions-Salze und Metallsalz-Komplexe.

2. Dichlorcyclopropylalkyl-hydroxyalkyl-azol-Derivate der Formel (I), in denen

0203440

R für gegebenenfalls durch Alkyl mit 1 bis 3 Kohlenstoffatomen substituiertes Cycloalkyl mit 3 bis 7 Kohlenstoffatomen oder für Aryl mit 6 bis 10 Kohlenstoffatomen steht, wobei jeder dieser Aryl-Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil Hydroximinoalkyl mit 1 oder 2 Kohlenstoffatomen, Alkoximinoalkyl mit 1 oder 2 Kohlenstoffatomen im Alkoxyteil und 1 oder 2 Kohlenstoffatomen im Alkylteil oder Phenyl, welches seinerseits durch Halogen und/oder Alkyl mit 1 bis 4 Kohlenstoffatomen substituiert sein kann, oder

R für die Gruppierungen

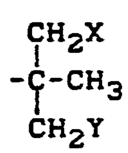 und 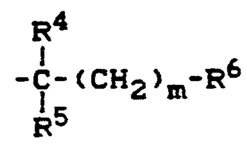 steht, worin

X für Fluor oder Chlor steht,

Y für Wasserstoff, Fluor oder Chlor steht,

$R^4$ für geradkettiges oder verzweigtes Alkyl mit 1 bis 4 Kohlenstoffatomen steht,

$R^5$ für geradkettiges oder verzweigtes Alkyl mit 1 bis 4 Kohlenstoffatomen steht,

$R^6$ für geradkettiges oder verzweigtes Alkyl mit 1 bis 4 Kohlenstoffatomen, für Halogenalkyl mit 2 oder 3 Kohlenstoffatomen und 1 bis 5 gleichen oder verschiedenen Halogenatomen, für Alkenyl mit 2 bis 4 Kohlenstoffatomen, für Alkinyl mit 2 bis 4 Kohlenstoffatomen, für gegebenenfalls einfach bis dreifach, gleichartig oder verschieden durch Halogen, Alkyl mit 1 bis 4 Kohlenstoffatomen und/oder Alkoxy mit 1 bis 4 Kohlenstoffatomen substituiertes Cycloalkyl mit 3 bis 7 Kohlenstoffatomen, ferner für Phenyl, Phenoxy und Phenylthio steht, wobei jeder dieser Phenylreste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen,

Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil, Hydroximinoalkyl mit 1 oder 2 Kohlenstoffatomen, Alkoximinoalkyl mit 1 oder 2 Kohlenstoffatomen im Alkoxyteil und 1 oder 2 Kohlenstoffatomen im Alkylteil oder Phenyl, welches seinerseits durch Halogen und/ oder Alkyl mit 1 bis 4 Kohlenstoffatomen substituiert sein kann, und $R^6$ außerdem für die Gruppierung

$R^7$-O-N=CH- steht,

in welcher

$R^7$ für geradkettiges oder verzweigtes Alkyl mit 1 bis 4 Kohlenstoffatomen, Alkenyl mit 2 bis 4 Kohlenstoffatomen oder für Benzyl steht, welches im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen,

Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil, Hydroximinoalkyl mit 1 oder 2 Kohlenstoffatomen, Alkoximinoalkyl mit 1 oder 2 Kohlenstoffatomen im Alkoxyteil und 1 oder 2 Kohlenstoffatomen im Alkylteil oder Phenyl, welches seinerseits durch Halogen und/oder Alkyl mit 1 bis 4 Kohlenstoffatomen substituiert sein kann, und

m für die Zahlen 0, 1 oder 2 steht,

$R^1$ für Wasserstoff oder Methyl steht,

$R^2$ für Wasserstoff, Methyl oder Chlor steht,

$R^3$ für Wasserstoff, Methyl oder Chlor steht,

n für die Zahlen 1 oder 2 steht und

Z für ein Stickstoffatom oder die CH-Gruppe steht.

3. Verfahren zur Herstellung von Dichlorcyclopropylalkyl-hydroxyalkyl-azol-Derivaten der Formel

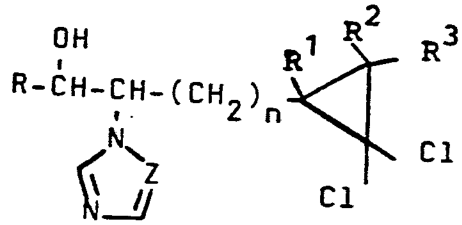 (I)

in welcher

R für gegebenenfalls substituiertes Cycloalkyl oder gegebenenfalls substituiertes Aryl sowie für die Gruppierungen

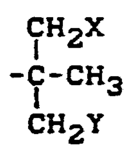 und 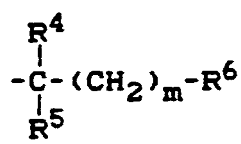 steht, wobei

X für Halogen steht,

Y für Wasserstoff oder Halogen steht,

$R^4$ und $R^5$ gleich oder verschieden sind und für Alkyl stehen,

$R^6$ für Alkyl, Halogenalkyl mit mehr als 2 Kohlenstoffatomen, Alkenyl, Alkinyl, gegebenenfalls substituiertes Cycloalkyl, gegebenenfalls substituiertes Aryl, gegebenenfalls substituiertes Aryloxy, gegebenenfalls substituiertes Arylthio oder für die Gruppierung

$R^7$-O-N=CH- steht,

in welcher

$R^7$ für Alkyl, Alkenyl oder gegebenenfalls substituiertes Benzyl steht,

und

m für die Zahlen 0, 1 oder 2 steht,

$R^1$,$R^2$ und $R^3$ unabhängig voneinander für Wasserstoff, Methyl oder Chlor stehen,

n für die Zahlen 1 oder 2 steht und

Z für ein Stickstoffatom oder die CH-Gruppe steht,

sowie von deren Säureadditions-Salzen und Metallsalz-Komplexen, dadurch gekennzeichnet, daß man Azolyl-keton-Derivate der Formel

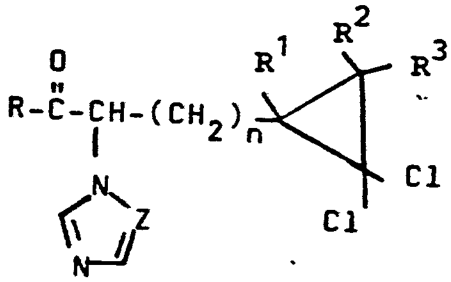

(II)

in welcher

$R, R^1, R^2, R^3$, n und Z die oben angegebenen Bedeutungen haben,

reduziert und gegebenenfalls anschließend eine Säure oder ein Metallsalz addiert.

4. Fungizide Mittel, gekennzeichnet durch einen Gehalt an mindestens einem Dichlorcyclopropylalkyl-hydroxyalkyl-azol-Derivat der Formel (I) bzw. einem Säureadditions-Salz oder Metallsalz-Komplex eines Dichlorcyclopropylalkyl-hydroxyalkyl-azol-Derivates der Formel (I).

5. Verwendung von Dichlorcyclopropylalkyl-hydroxyalkyl-azol-Derivaten der Formel (I) bzw. von deren Säureadditions-Salzen oder Metallsalz-Komplexen zur Bekämpfung von Pilzen.

6. Verfahren zur Bekämpfung von Pilzen, dadurch gekennzeichnet, daß man Dichlorcyclopropylalkyl-hydroxyalkyl-azol-Derivate der Formel (I) bzw. deren Säureadditions-Salze oder Metallsalz-Komplexe auf Pilze und/oder deren Lebensraum ausbringt.

7. Verfahren zur Herstellung von fungiziden Mitteln, dadurch gekennzeichnet, daß man Dichlorcyclopropylalkyl-hydroxyalkyl-azol-Derivate der Formel (I) bzw. deren Säureadditions-Salze oder Metallsalz-Komplexe mit Streckmitteln und/oder oberflächenaktiven Stoffen vermischt.

8. Azolyl-keton-Derivate der Formel

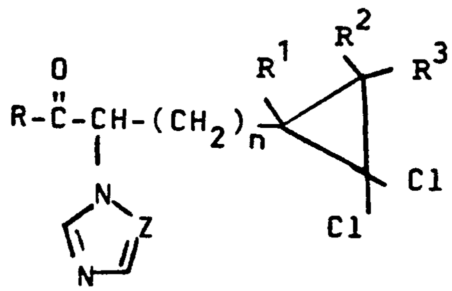 (II)

in welcher

R für gegebenenfalls substituiertes Cycloalkyl oder gegebenenfalls substituiertes Aryl sowie für die Gruppierungen

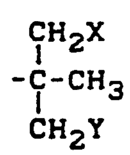 und 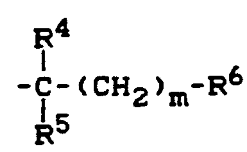 steht, wobei

X für Halogen steht,

Y für Wasserstoff oder Halogen steht,

$R^4$ und $R^5$ gleich oder verschieden sind und für Alkyl stehen,

$R^6$ für Alkyl, Halogenalkyl mit mehr als 2 Kohlenstoffatomen, Alkenyl, Alkinyl,

gegebenenfalls substituiertes Cycloalkyl, gegebenenfalls substituiertes Aryl, gegebenenfalls substituiertes Aryloxy, gegebenenfalls substituiertes Arylthio oder für die Gruppierung

$R^7$-O-N=CH- steht,

in welcher

$R^7$ für Alkyl, Alkenyl oder gegebenenfalls substituiertes Benzyl steht,

und

m für die Zahlen 0, 1 oder 2 steht,

$R^1$,$R^2$ und $R^3$ unabhängig voneinander für Wasserstoff, Methyl oder Chlor stehen,

n für die Zahlen 1 oder 2 steht und

Z für ein Stickstoffatom oder die CH-Gruppe steht.

9. Verfahren zur Herstellung von Azolyl-keton-Derivaten der Formel

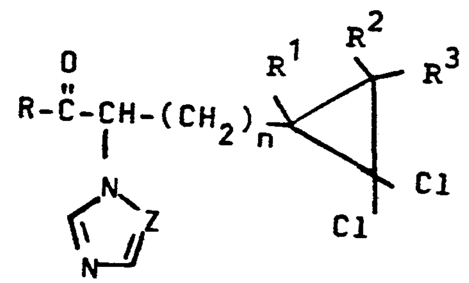

(II)

in welcher

R für gegebenenfalls substituiertes Cycloalkyl oder gegebenenfalls substituiertes Aryl sowie für die Gruppierungen

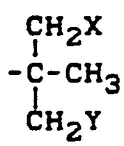 und 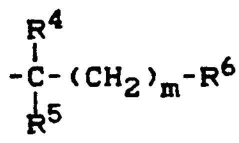 steht, wobei

X für Halogen steht,

Y für Wasserstoff oder Halogen steht,

$R^4$ und $R^5$ gleich oder verschieden sind und für Alkyl stehen,

$R^6$ für Alkyl, Halogenalkyl mit mehr als 2 Kohlenstoffatomen, Alkenyl, Alkinyl, gegebenenfalls substituiertes Cycloalkyl, gegebenenfalls substituiertes Aryl, gegebenenfalls substituiertes Aryloxy, gegebenenfalls substituiertes Arylthio oder für die Gruppierung

$R^7$-O-N=CH- steht,

in welcher

$R^7$ für Alkyl, Alkenyl oder gegebenenfalls substituiertes Benzyl steht,

und

m für die Zahlen 0, 1 oder 2 steht,

$R^1$,$R^2$ und $R^3$ unabhängig voneinander für Wasserstoff, Methyl oder Chlor stehen,

n für die Zahlen 1 oder 2 steht und

Z für ein Stickstoffatom oder die CH-Gruppe steht,

dadurch gekennzeichnet, daß man Azolylketone der Formel

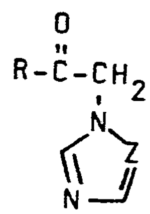

(III)

in welcher

R und Z die oben angegebene Bedeutung haben,

mit Verbindungen der Formel

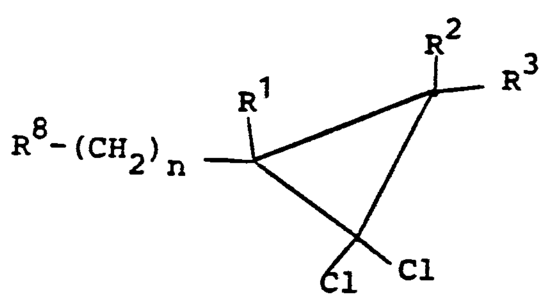

(IV)

in welcher

$R^1$, $R^2$, $R^3$ und n die oben angegebene Bedeutung haben

und

$R^8$ für eine elektronenziehende Abgangsgruppe steht,

in Gegenwart eines inerten Verdünnungsmittels und in Gegenwart eines Säurebindemittels umsetzt.